(19) Europäisches Patentamt
European Patent Office
Office européen des brevets

(11) Veröffentlichungsnummer: **0 167 954 B1**

(12) **EUROPÄISCHE PATENTSCHRIFT**

(45) Veröffentlichungstag der Patentschrift: **27.09.89**

(21) Anmeldenummer: **85108137.2**

(22) Anmeldetag: **01.07.85**

(51) Int. Cl.⁴: **C 07 H 15/252**, A 61 K 31/70, C 07 C 50/36, A 61 K 31/12, C 12 P 19/56, C 12 P 7/26 // (C12P19/56, C12R1:465),(C12P7/26, C12R1:465)

(54) **1-Hydroxy-Cytorhodine, ein mikrobiologisches Verfahren zu ihrer Herstellung und ihre Verwendung als Cytostatika.**

(30) Priorität: **10.07.84 DE 3425357**

(43) Veröffentlichungstag der Anmeldung: **15.01.86 Patentblatt 86/3**

(45) Bekanntmachung des Hinweises auf die Patenterteilung: **27.09.89 Patentblatt 89/39**

(84) Benannte Vertragsstaaten: **AT BE CH DE FR GB IT LI LU NL SE**

(56) Entgegenhaltungen:
**EP-A- 0 022 574**
**EP-A- 0 131 942**
**EP-A- 0 203 329**

**TETRAHEDRON LETTERS, Band 45, 1968, Seiten 4719-4724, Pergamon Press Ltd., Oxford, GB. H. BROCKMANN et al.: "Die absolute Konfiguration der Anthracyclinone"**
**CHEMICAL ABSTRACTS, Band 87, Nr. 17, 24. Oktober 1977, Seite 690, Ref. Nr. 134839k, Columbus, Ohio, US**
**JOURNAL OF ANTIBIOTICS, Band 34, Nr. 12, Dezember 1981, Seiten 1596-1607, Tokyo, JP. Y. MATSUZAWA et al.: "Structure-activity relationships of anthracyclines relative to cytotoxicity and effects on macromolecular synthesis in L1210 leukemia cells"**

(73) Patentinhaber: **HOECHST AKTIENGESELLSCHAFT, Postfach 80 03 20, D-6230 Frankfurt am Main 80 (DE)**

(72) Erfinder: **Aretz, Werner, Dr., Am Krummorgen 2, D-6233 Kelkheim (Taunus) (DE)**
Erfinder: **Berscheid, Hans Gerd, Dr., Rheinlandstrasse 21, D-6231 Schwalbach (DE)**
Erfinder: **Fehlhaber, Hans-Wolfram, Dr., Thomas-Mann-Strasse 5a, D-6270 Idstein/Taunus (DE)**
Erfinder: **Böttger, Dirk, Dr., Bahnstrasse 4, D-6237 Liederbach (DE)**
Erfinder: **Kraemer, Hans Peter, Dr., Birkenweg 16, D-3550 Marburg (DE)**

**EP 0 167 954 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft 1-Hydroxy-Cytorhodine der Formel I .

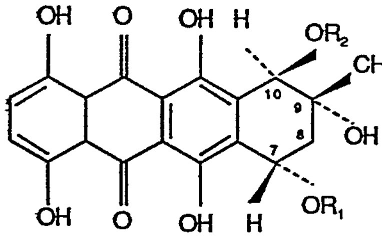

I

worin

a) $R_1$ und $R_2$ gleich sind und jeweils die Zuckerkombination Roa-Rod-Rod oder Roa-dF=CinB darstellen, oder

b) $R_1$ und $R_2$ verschieden sind und jeweils die Zuckerkombination Roa-Rod-Rod und Roa-dF-Rod darstellen, oder

c) $R_1$ und $R_2$ verschieden sind, wobei $R_1$ die Zukkerkombination Roa-dF-CinA und $R_2$ die Zuckerkombination Roa-Rod-Rod oder Roa-Rod-Acu darstellt oder $R_1$ die Zuckerkombination Roa-Rod-Acu und $R_2$ die Zuckerkombination Roa-Rod-Rod darstellt,

sowie deren physiologisch unbedenklichen Säureadditionssalze.

Die vorliegende Erfindung betrifft ferner ein Verfahren zur Herstellung von Verbindungen der Formel I, das dadurch gekennzeichnet ist, dass man den Stamm Streptomyces purpurascens - DSM 2658 bei einem pH-Wert von 6,5-8,5, einer Temperatur von 24-40°C und einer hohen Belüftungsrate in einem geeigneten Nährmedium fermentiert und die Verbindungen der Formel I aus der Kulturflüssigkeit und dem Mycel nach üblichen Methoden isoliert.

Die Isolierung und Identifizierung von Streptomyces purpurascens - DSM 2658 ist in der deutschen Patentanmeldung P 3 323 025.0 beschrieben. Der Mikroorganismus wurde am 24.5.1983 bei der DSM (Deutsche Sammlung von Mikroorganismen) in D-3400 Göttingen (Grisebachstr. 8) hinterlegt.

In der genannten Patentanmeldung wird auch ein Verfahren zur Herstellung von 1-Deshydroxy-Anthracyclin-Derivaten der Formel 1, den sogenannten Cytorhodinen, durch Fermentation des Stammes Streptomyces purpurascens - DSM 2658 unter aeroben Bedingungen beschrieben.

Es wurde nun überraschenderweise gefunden, dass bei Erhöhung der Sauerstoffversorgung bei der Fermentation 1-Hydroxy-Cytorhodine gebildet werden. Diese neuen vor allem cytostatisch wirksamen Verbindungen gehören zur Gruppe der 1-Hydroxy-Anthracycline, von denen die folgenden Vertreter bereits beschrieben sind:

1-Hydroxy-β-Rhodomycine - auch β-Iso-Rhodomycine genannt -: z.B. Violamycine $A_2$ und BI-1 (W. Fleck et al., Z. Allgem. Mikrobiologie 14, 551 (1974) und β-Isorhodomycine (H. Brockmann et al., Chem. Ber. 98, 3145 (1965).

ε-Pyrromycine (Cinerubine): z.B. Cinerubin A (W. Keller-Schierlein, Antimicrob. Agents Chemother. 68 (1970)), verschiedene Rhodirubine (H. Umezawa et al., J. Antib. 30, 616 (1977) und die Aclacinomycine $A_2$, $B_2$, $M_2$, $S_2$ und $T_2$ (T. Oki et al., J. Antib. 28, 830 (1975) und 32, 801 (1979), sowie Musetta-, Marcello-, Rudolphomycin u.ä. (D.E. Nettleton et al., J. Nat. Prod. 43, 242 (1980)).

Aus J. Antibiotics 34 (1981), No. 12, S. 1596-1607 sind cytostatisch wirksame Mono-trisaccharid-glykoside des 1-Hydroxyanthrachinons, d.h. solche, die entweder in 7- oder in 10-Stellung des Aglykons ein Trisaccharidmolekül tragen, bekannt.

Das erfindungsgemässe Verfahren zur Herstellung von Verbindungen der allgemeinen Formel I wird durch Fermentation von Streptomyces purpurascens - DSM 2658 bei einem pH-Wert von 6,5-8,5 und einer Temperatur von 24-40°C unter aeroben Bedingungen bei einer genügend hohen Sauerstoffversorgung z.B. durch eine hohe Belüftungsrate in einem Kohlenstoff- und Stickstoffquellen sowie anorganische Nährsalze und Spurenelemente enthaltenden Nährmedium und anschliessende Isolierung der Verbindung aus der Kulturflüssigkeit und dem Mycel nach üblichen Methoden wie nachfolgend beschrieben, durchgeführt.

Als Kohlenstoffquellen eignen sich Glucose, Stärke, Dextrin und Glycerin. Geeignete Stickstoffquellen sind Sojamehl, Hefeextrakt, Fleischextrakt, Malzextrakt, Maisquellwasser, Pepton oder Casein. Als anorganische Nährsalze eignen sich z.B. Natriumchlorid, Magnesiumsulfat oder Calciumkarbonat. Als Spurenelemente kann man Eisen, Magnesium, Kupfer, Zink und Kobalt verwenden.

Streptomyces purpurascens - DSM 2658 kann bei Temperaturen von 24-40°C bei einem pH von 6,5-8,5 und einer Belüftungsrate von 0,8-1,2 vvm fermentiert werden. Vorzugsweise erfolgt die Fermentation des Streptomyces purpurascens - DSM 2658 unter aeroben Bedingungen bei 30°C und bei einem pH-Wert von 7,0. Nach 70-130, vorzugsweise 90-110 Stunden, wenn die höchste Ausbeute erreicht ist, bricht man die Fermentation ab. Vorzugsweise kann es sich bei der Fermentation um eine Submers-Fermentation handeln.

Der Fortschritt der Fermentation und die Bildung der Anthracyclinverbindungen kann anhand der antibakteriellen Wirksamkeit gegen S. aureus 209 P und Bac. subt. sowie direkt durch Extraktion der gesamten Kulturlösung (Mycel und Kulturfiltrat) mit einem organischen Lösungsmittel und Messung der Absorptionsintensität der violetten Verbindungen bei 495, 525 und 568 nm verfolgt werden. Als organisches Lösungsmittel verwendet man vorzugsweise Äthylacetat.

Die Anthracyclinverbindungen im Kulturfiltrat und im Mycel werden gemäss dem Schema im Schaubild I (vgl. auch Beispiel 3) der vorliegenden Anmeldung isoliert.

Die Anthracyclinverbindungen im Mycel werden mit einem organischen Lösungsmittel, vorzugsweise mit wässrigem Aceton, das auf einen

pH-Wert von 3,5 eingestellt wurde, extrahiert. Nach Entfernung des Acetons stellt man den pH-Wert der wässrigen Phase auf 7,5 ein und extrahiert dann bei einem pH-Wert von 7,5 mit einem organischen Lösungsmittel wie Butylacetat, Äthylacetat oder Chloroform, vorzugsweise mit Äthylacetat. Die Äthylacetatextrakte aus dem Mycel und dem Kulturfiltrat werden vereinigt oder separat aufgearbeitet, konzentriert und mit einem Acetatpuffer (3,5 pH) extrahiert. Die wässrige Phase wird mit einem organischen Lösungsmittel wie Benzol oder Toluol extrahiert. Auf dieser Stufe teilt sich das Gemisch der Anthracyclin-Derivate in zwei Fraktionen auf. Das in der Toluolphase verbleibende Gemisch bezeichnet man als Fraktion A, das in der wässrigen Phase verbleibende Glykosidgemisch bezeichnet man als Fraktion B.

Die Fraktion B wird weiter gereinigt gemäss Schaubild II. Wie im Schaubild II dargestellt, erhält man aus der Fraktion B die 1-Hydroxy-Cytorhodine A, B, C und P und das Gemisch N + O (1:1). Bei den Fraktionen I, II, III und IV handelt es sich um nicht aufgetrennte Mischungen aus Anthracyclinverbindungen der Formel I.

Die aus der Kulturlösung von Streptomyces purpurascens – DSM 2658 isolierten und gemäss Schaubild I gereinigten Verbindungen haben die allgemeine Formel I.

Schaubild I

Isolierung des antibiotischen Komplexes aus Streptomyces purpurascens – DSM 2568

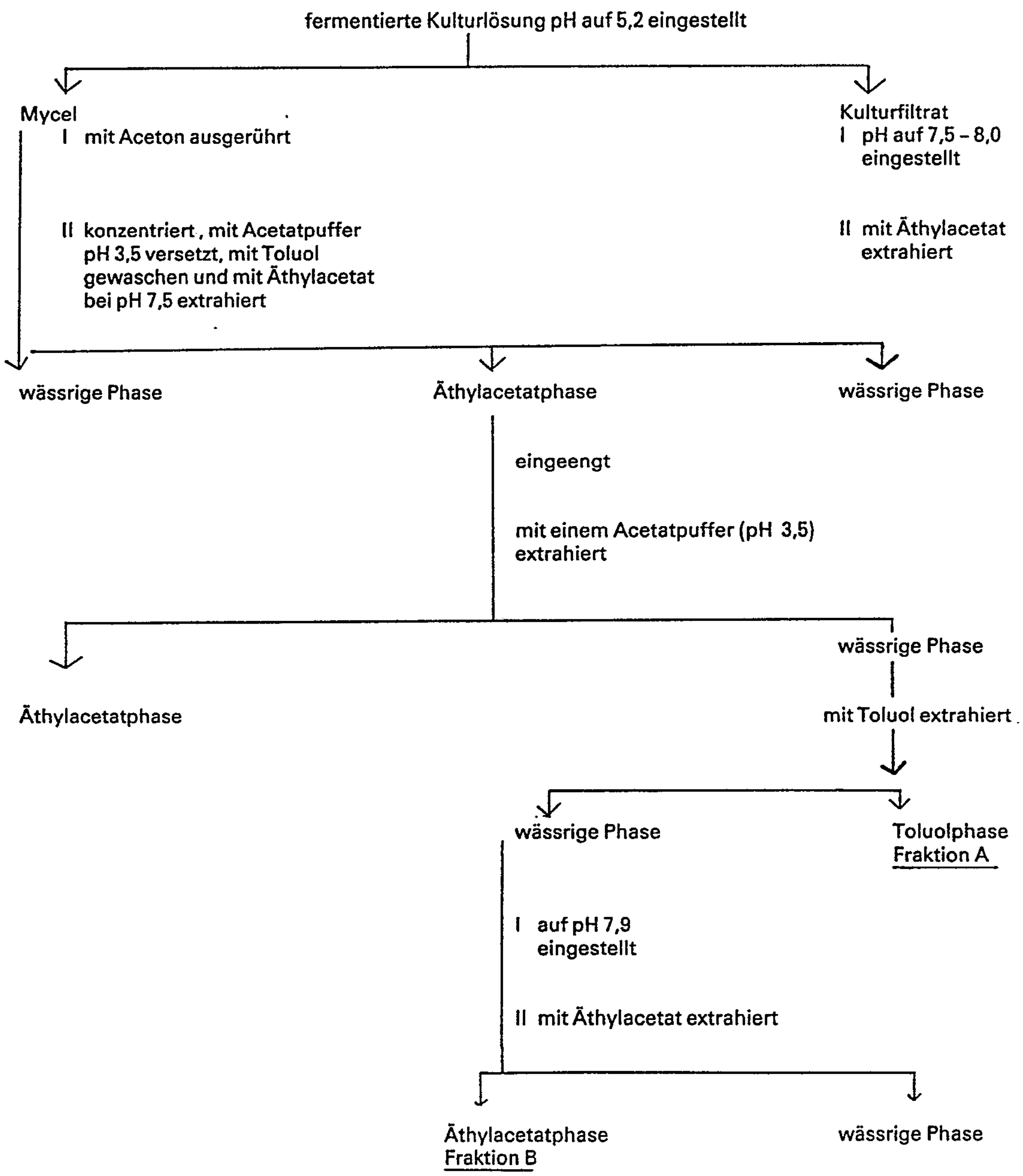

Schaubild II

Aufarbeitung der wässrigen Phase

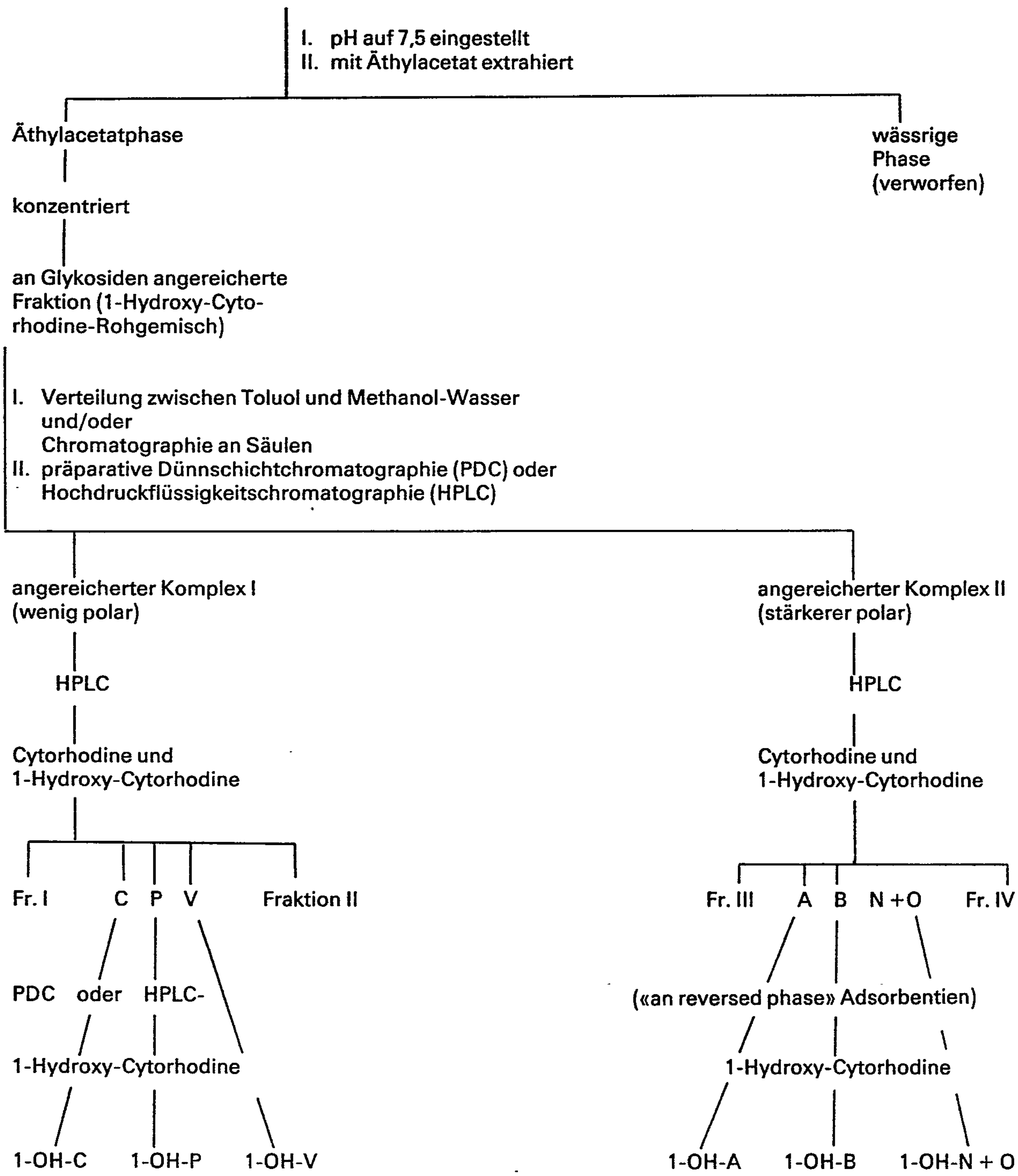


Die bevorzugten erfindungsgemässen Verbindungen haben die in Formel I

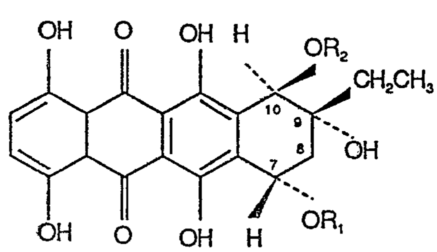

I

dargestellte Struktur, wobei $R_1$ und $R_2$ die folgenden Bedeutungen haben:

| Verbindung | $R_1$ | $R_2$ |
|---|---|---|
| 1-Hydroxy-Cytorhodin V | Roa–Rod–Acu (Formel III) | Roa–Rod–Rod (Formel VI) |
| 1-Hydroxy-Cytorhodin C | Roa–dF=Cin B (Formel IV) | Roa–dF=Cin B (Formel IV) |
| 1-Hydroxy-Cytorhodin B | Roa–dF–Cin A (Formel V) | Roa–Rod–Rod (Formel VI) |
| 1-Hydroxy-Cytorhodin A | Roa–Rod–Rod (Formel VI) | Roa–Rod–Rod (Formel VI) |
| 1-Hydroxy-Cytorhodin N | Roa–Rod–Rod (Formel VI) | Roa–dF–Rod (Formel VII) |
| 1-Hydroxy-Cytorhodin O | Roa–dF–Rod (Formel VII) | Roa–Rod–Rod (Formel VI) |
| 1-Hydroxy-Cytorhodin P | Roa–dF–Cin A (Formel VII) | Roa–Rod–Acu (Formel V) |

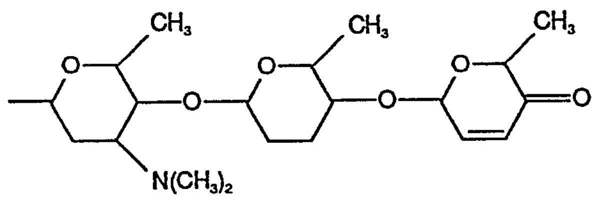

Roa-Rod-Acu

(III)

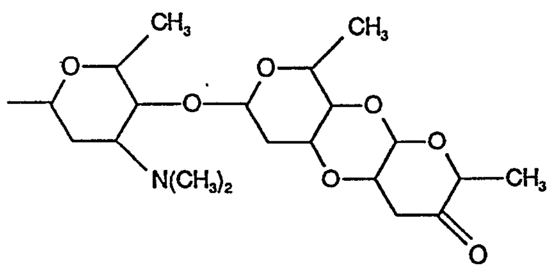

Roa-dF=Cin B

(IV)

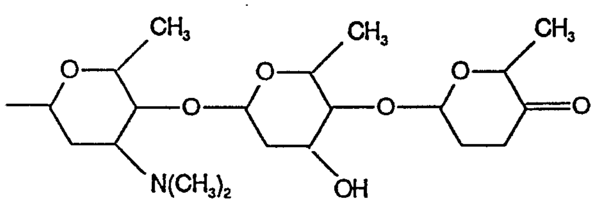

Roa-dF-Cin A

(V)

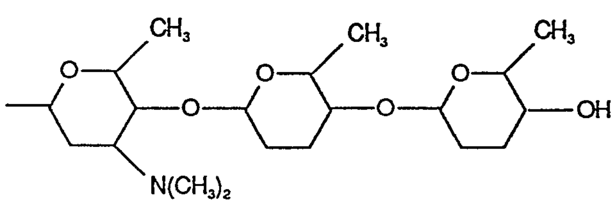

Roa-Rod-Rod

(VI)

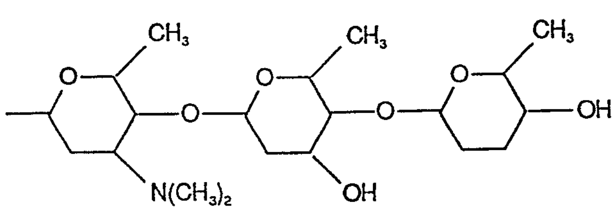

Rod-dF-Rod

(VII)

Die erfindungsgemässen Anthracyclinverbindungen zeichnen sich durch eine starke Wirkung gegen grampositive Bakterien sowie eine ausgeprägte cytostatische Wirkung aus.

Die vorliegende Erfindung wird durch die nachfolgenden Beispiele näher erläutert:

Beispiel 1

Herstellung von 1-Hydroxy-Cytorhodinen durch Fermentation von Streptomyces purpurascens - DSM 2658.

Streptomyces purpurascens - DSM 2658 wurde auf Hefe-Malz-Agar der folgenden Zusammensetzungen gegeben:

| | |
|---|---|
| Malzextrakt | 10 g |
| Hefeextrakt | 4 g |
| Glukose | 4 g |
| Agar | 15 g |
| dest. Wasser | 1 l |
| pH | 7,0 |

Dazu wurde das Medium auf Roux-Flaschen verteilt und 30 Minuten bei 121 °C sterilisiert, abgekühlt, mit einer Sporensuspension beimpft und 7–14 Tage bei 25 °C inkubiert. Danach konnte man ein gutes Wachstum und eine gute Sporenbildung feststellen. Von dieser Stammkultur wurde eine Sporensuspension mit $10^7$ Sporen/ml in steriler NaCl (0,8%ig) hergestellt. 10 ml dienten als Inokulum von 500 ml fogenden Vorkulturmediums:

| | |
|---|---|
| Glukose | 15 g |
| Sojamehl | 15 g |
| Cornsteep (fest) | 5 g |
| $CaCO_3$ | 2 g |
| NaCl | 5 g |
| dest. Wasser | 1 l |
| pH | 7,0 |

Je 500 ml des obigen Mediums wurden auf 2000-ml-Erlenmeyerkolben verteilt und 30 Min. bei 121 °C sterilisiert. Die beimpften Kolben wurden 2 Tage bei 25 °C und 200 Upm auf einem Rotationsschüttler inkubiert.

500 ml der gewachsenen Vorkultur wurden als Inokulum für folgende Hauptkultur verwendet:

| | |
|---|---|
| Glukose | 20 g |
| Malzextrakt | 10 g |
| Hefeextrakt | 4 g |
| dest. Wasser | 1 l |
| pH | 6,8 |

9 Liter des obigen Mediums wurden in einen 12-l-Fermenter gegeben und 50 Min. bei 121 °C sterilisiert. Die Fermentation wurde bei 28 °C, einer Rührergeschwindigkeit von 600 Upm und einer Belüftungsrate von 8–9 l Luft/Min. durchgeführt.

Die Fermentationsdauer betrug 90–110 Stunden. Zur Feststellung des Aberntezeitpunktes wurde die Ausbildung des Absorptionsmaximums bei 568 nm verfolgt. Dazu wurden Proben der Kulturlösung mit Äthylacetat extrahiert und das Absorptionsspektrum zwischen 400 und 600 nm aufgenommen.

Beispiel 2

Fermentation von Streptomyces purpurascens - DSM 2658 in grösserem Massstab.

Gemäss Beispiel 1 wurde Streptomyces purpurascens - DSM 2658 in 2000-ml-Erlenmeyerkolben mit 500 ml Vorkulturmedium angezogen und nach 2 Tagen in einen 30-l-Fermenter mit 20-l-Vorkulturmedium überimpft. Die Fermentation erfolgte bei 28 °C unter Rühren bei 160–180 Upm mit einer Belüftungsrate von 6–7 l/Min. Nach 48 Stunden diente diese Vorkultur als Inokulum für einen 300-l-Fermenter mit 200-l-Hauptkulturmedium (Beispiel 1). Die Fermentation erfolgte 90–110 Stunden bei 28 °C, einer Rührerumfangsgeschwindigkeit von 3,5 m/sec. und einer Belüftungsrate von 1 vvm (200 l/Min.). Die Erntekriterien entsprechen denen in Beispiel 1.

Beispiel 3

Isolierung des Rohgemisches der Anthracyclinverbindungen.

190 l KL wurden mit 0,5% Formalin versetzt und mit Essigsäure auf pH = 5,2 gestellt. Das Mycel wurde mit den üblichen Methoden (Feststoffseparator, Filterpresse) abgetrennt und mehrmals mit wenig Wasser gewaschen.

Das Kulturfiltrat (ca. 185 l) wurde mit dem Waschwasser vereinigt, mit NaOH auf pH = 7,5–8,0 gestellt und 2× mit je 50 l Ethylacetat extrahiert. Die wässrige Phase wurde verworfen. Die organischen Phasen wurden vereinigt, im Vakuum auf 8 l eingeengt und dann 4× mit 3 l Natriumacetat-Pufferlösung (pH 3,5) extrahiert. Die vereinigten Pufferlösungen wurden anschliessend 3× mit 3 l Toluol gewaschen.

Das abgetrennte Mycel (ca. 3,4 kg) wurde 3× mit 15 l Aceton ausgerührt, die abdekantierte Acetonlösung im Vakuum eingeengt, mit 5 l einer Na-Acetat-Pufferlösung vom pH 3,5 versetzt und 3× mit 2,5 l Toluol gewaschen.

Die wässrige Pufferphase der Kulturfiltrataufarbeitung wurde mit NaOH auf pH = 7,9 gestellt und 3× mit 3 l Ethylacetat extrahiert. Die vereinigten Ethylacetatextrakte wurden im Vakuum eingeengt.

Die wässrige Pufferphase der Mycelaufarbeitung wurde mit NaOH auf pH = 8,0 gestellt und 3× mit je 2,5 l Ethylacetat extrahiert. Die vereinigten organischen Extrakte wurden im Vakuum eingeengt.

Die verbleibenden wässrigen Phasen wurden verworfen.

Beispiel 4

Isolierung von 1-Hydroxy-Cytorhodin A durch «reversed phase» HPLC.

Eine stark violette Charge von Cytorhodin A aus rohem polarem Cytorhodin-Gemisch (Komplex II), gewonnen durch übliche Säulenchromatographie an Kieselgel mit essigsäurehaltiger mobiler Phase und nachfolgende, weitere Anreicherung durch präparative HPLC an $SiO_2$, wurde durch präparative HPLC an einem «reversed phase» Adsorbens weiter aufgetrennt. So wurden z.B. 250 mg in 4 ml einer mobilen Phase $CHCl_3$-Methanol-10% Ammonacetat in Wasser 150:1050:375 gelöst und auf eine Stahlsäule (3,2×25 cm), mit ca. 120 g LiChrosorb® RP-18, 25–40 μ (Merck) unter Druck gefüllt gegeben und bei einem Fluss von 4 ml/Min. chromatographiert.

Die Trennung wurde mit zwei hintereinander geschalteten Durchflussphotometern bei den Wellenlängen 490 und 560 nm verfolgt. Nach Zusammenfassung und üblicher Aufarbeitung jeweils der roten und der violetten Fraktionen wurde folgendes Ergebnis erreicht.

Fr. 88–108 62 mg Cytorhodin A (nach $^1$H-NMR)
Fr. 109–120 46 mg 1-Hydroxy-Cytorhodin A (ca. 80–90% Reinheit)

Etwa 150 mg von auf obige Weise gewonnenem 1-Hydroxy-Cytorhodin A wurde durch erneute präparative HPLC an RP-18, 25–40 μm unter obigen Bedingungen weiter gereinigt. Erhalten wurden 60 mg 1-Hydroxy-Cytorhodin A mit weniger als 2% restlichem Cytorhodin A.

1-OH-A: $^1$H-NMR
Adsorptionsspektrum (nm)
a) 240 (4,67) 298 (3,73) 522 (4,06) 550 (4,07) 561 (4,10)
b) 240 (4,75) 255 (SCH) 270 (4,37) 522 (4,30) 550 (4,29) 561 (4,30)
c) 243 (4,69) 276 (3,81) 589 (4,30) 635 (4,44)

$C_{60}H_{88}N_2O_{21}$, M ber.: 1172 (FAB-MS bestätigt).

Beispiel 5

Isolierung von 1-Hydroxy-Cytorhodin V durch präparative Mitteldruck- und Schichtchromatographie.

7 g rohes Cytorhodingemisch, gewonnen durch Extraktion aus der Kulturlösung wie im Schema I beschrieben, wurden an 620 g Kieselgel 31 μm (Grace) in zwei radial komprimierten Silica-Patronen (Waters, Prep LC/System 500®R) mit dem Laufmittelgemisch $CHCl_3$/Methanol/96%ige Essigsäure/Wasser = 80:10:10:2 chromatographiert. Die Probe wurde in 65 ml der mobilen Phase auf die äquilibrierte Säule gegeben und bei einem Fluss von 25 ml/Min. in Fraktionen zu 23 ml aufgetrennt. Die Beurteilung erfolgte dünnschichtchromatographisch und mit Hilfe der analytischen HPLC.

In Fraktion 29–34 fielen 195 mg eines Gemisches aus Cytorhodin V und 1-Hydroxy-Cytorhodin V in einer Reinheit von 80% an ($R_f$ 0,38 im System A).

60 mg dieser Probe wurden durch präparative Dünnschichtchromatographie (Merck, DC-Fertigplatten Kieselgel 60) weiter gereinigt.

Die Elution der herausgeschabten Banden erfolgte mit $CHCl_3$/Methanol 1:1. Die Elutionslösung wurde mit wässriger Dinatriumhydrogenphosphat-Lösung neutralisiert und mit Wasser bis zur Abtrennung der $CHCl_3$-Phase versetzt. Die Chloroformphase wurde separiert, einmal mit wenig Wasser gewaschen, über wasserfreiem $Na_2SO_4$ getrocknet und eingedampft. Nach Aufnehmen in wenig Chloroform wurde mit der 10fachen Volumenmenge Petroläther gefällt und der Niederschlag i.V. getrocknet. Es wurden neben 12 mg Cytorhodin V 17 mg 1-Hydroxy-Cytorhodin V erhalten.

V: durch $^1$H-NMR identifiziert
$C_{60}H_{84}N_2O_{20}$, M ber, 1152 (FAB-MS bestätigt).

1-OH-V: $^1$H-NMR
Absorptionsspektrum (nm)
a) 237 (4,54) 295 (3,72) 522 (4,03) 548 (3,86) 561 (3,68)
b) 237 (4,57) 295 (3,79) 522 (4,10) 548 (3,93) 561 (3,93)
c) 244 (4,46) 280 (3,76) 590 (3,99) 630 (4,04)
$C_{60}H_{84}N_2O_{21}$, M ber.: 1168 (FAB-MS bestätigt).

Beispiel 6

Isolierung von 1-Hydroxy-Cytorhodin B durch «reversed phase» HPLC.

Eine durch übliche präparative Säulenchromatographie an Kieselgel (essigsäurehaltige mobile Phase) gewonnene violette Charge von Cytorhodin B wurde an einer mit 30 g 10 μ Lichrosorb® RP-18 (Merck) gefüllten Stahlsäule (1,6×25 cm) unter Druck chromatographiert. Dazu wurden 120 mg in 1 ml der mobilen Phase $CHCl_3$-MeOH-10% Ammonacetat in Wasser 150:1050:375 gelöst und injiziert. Bei einem Fluss von 4 ml/Min. wurden Fraktionen von 4 ml aufgefangen und durch Messung der Extinktion bei 490 und 570 nm beurteilt.

Zusammengehörende Fraktionen wurden vereinigt und wie üblich aufgearbeitet.

Es wurden erhalten:
Fr. 1–32 15 mg Cytorhodin B
Fr. 38–50 10 mg 1-Hydroxy-Cytorhodin B

B: Identifizierung durch $^1$H-NMR, Absorptionsspektrum und FAB-MS

1-OH-B: $^1$H-NMR
Absorptionsspektrum (nm)
a) 259 (4,42) 290 (3,66) 522 (3,95) 549 (3,82) 562 (3,85)
b) 238 (4,45) 298 (3,57) 522 (4,03) 549 (3,91) 562 (3,93)
c) 244 (4,41) 280 (3,62) 590 (3,93) 632 (4,01)
$C_{60}H_{86}N_2O_{22}$, M ber.: 1186 (FAB-MS bestätigt).

Beispiel 7

Isolierung von 1-Hydroxy-Cytorhodin C durch «reversed phase» HPLC.

Ein durch übliche präparative Säulenchromatographie an Kieselgel gewonnenes rohes violettes Cytorhodin-C-haltiges Produkt (25 mg) wurde an einer mit 30 g 10 μm Lichrosorb® RP-18 (Merck) gefüllten Stahlsäule (1,6×25 cm) mit der

Mischung $CHCl_3$-Methanol-10% Ammonacetat in Wasser 150:1050:375 bei einem Fluss von 2 ml/Min. chromatographiert. Fraktionen von 2 ml wurden aufgefangen und nach Beurteilung durch Messung der Extinktionen bei 490 und 570 nm vereinigt. Nach üblicher Aufarbeitung wurden erhalten:

Fr. 80–142 10 mg Cytorhodin C
Fr. 159–175 7 mg 1-OH-Cytorhodin C

1-OH-C: $^1$H-NMR
Absorptionsspektrum (nm)
a) 240 (4,62) 296 (3,80) 523 (4,13) 550 (4,10) 562 (4,11)
b) 240 (4,64) 296 (3,80) 523 (4,21) 550 (4,15) 562 (4,16)
c) 243 (4,69) 270 (SCH) 300 (SCH) 590 (4,24) 635 (4,35)
$C_{60}H_{80}N_2O_{23}$, M ber.: 1196 (FAB-MS bestätigt).

Beispiel 8

Isolierung von 1-Hydroxy-Cytorhodin N + O durch «reversed phase» HPLC.

Durch übliche mehrstufige Säulenchromatographie an 31 µ Kieselgel, pH 7,5, und anschliessend an 10 µm LiChrosorb® RP-18 (s. a. Beispiel 4) wurde eine stark violette Charge eines sich nach DC und analytischer HPLC an Kieselgel mit System B wie Cytorhodin N + O verhaltenden Produktes gewonnen, die durch wiederholte präparative HPLC an «reversed phase»-Adsorbentien weiter in einen roten und einen violetten Anteil aufgetrennt werden konnte.

Dazu wurden z. B. 230 mg des violetten Produktes in 4 ml der mobilen Phase $CHCl_3$-Methanol-10% Ammonacetat in Wasser 150:1050:375 gelöst und an ca. 100 g 10 µm Lichrosorb® RP-18 (Merck) in einer Stahlsäule 3,2 × 25 cm unter Druck bei einem Fluss von 4 ml/Min. chromatographiert. Fraktionen von 8 ml wurden gesammelt und nach Beurteilung durch analytische «reversed phase» HPLC mit Doppelwellenlängendetektion bei 490 und 560 nm zusammengefasst. Erhalten wurden nach üblicher Aufarbeitung:

Fr. 70–85 97 mg Cytorhodin N + O (ca. 70–80% Rheinheit - nach $^1$H-NMR)
Fr. 86–95 40 mg 1-Hydroxy-Cytorhodin N + O (ca. 60% Reinheit)

Aus zwei ähnlichen chromatographischen Läufen wurden zusammen 73 mg 1-Hydroxy-Cytorhodin N + O gewonnen und in der obigen Weise rechromatographiert. Erhalten wurden 18 mg 1-Hydroxy-Cytorhodin N + O mit weniger als 10% restlichem Cytorhodin N + O.

1-OH-N + O: $^1$H-NMR
Absorptionsspektrum (nm)
a) 240 (4,62) 295 (3,77) 523 (4,16) 550 (4,09) 562 (4,11)
b) 240 (4,64) 295 (3,83) 523 (4,22) 550 (4,15) 562 (4,16)
c) 244 (4,64) 280 (SCH) 592 (4,19) 634 (4,28)
$C_{60}H_{88}N_2O_{22}$, M ber., 1188 (FAB-MS bestätigt). - Das isolierte Produkt ist ein 1:1-Gemisch der beiden stukturisomeren Komponenten 1-OH-N + 1-OH-O. Dies geht aus dem $^1$H-NMR-Spektrum, Abbauversuchen (Hydrogenolyse) und Bestimmung der einzelnen Zuckerbausteine nach Hydrogenolyse und Totalhydrolyse hervor.

Beispiel 9

Isolierung von 1-Hydroxy-Cytorhodin P durch «reserved phase» HPLC.

Durch übliche Säulenchromatographie an 31 µm Kieselgel pH 7,5 wurde ein stark violettes Produkt erhalten, dessen Hauptkomponente bei DC (System A) und analytischer HPLC an Kieselgel eine geringfügig höhere Retention als 1-Hydroxy-Cytorhodin C zeigte. Durch wiederholte präparative «reversed phase» HPLC konnte die neue Verbindung isoliert werden. Dazu wurden z.B. 175 mg des violetten Rohproduktes in 2,5 ml der mobilen Phase $CHCl_3$-Methanol 15% Ammonacetat in Wasser 500:1100:300 gelöst, und an ca. 100 g 10 µm LiChrosorb® RP-18 (Merck) in einer Stahlsäule 3,2 × 25 cm unter Druck bei einem Fluss von 4 ml/Min. chromatographiert. Das Eluat wurde in Fraktionen von 8 ml gesammelt und zusammengehörende Fraktionen nach Beurteilung durch analytische «reversed-phase»-HPLC mit Doppelwellenlängendetektion bei 490 und 560 nm vereinigt.

Erhalten wurden:

nach analytischer «reversed phase»-HPLC
Fr. 21–45 85 mg u. a. 1-Hydroxy-Cytorhodine C und V
Fr. 46–52 50 mg u. a. Cytorhodin P und nicht identifizierte stärker polare Verbindungen
Fr. 53 - 61 25 mg hauptsächlich 1-Hydroxy-Cytorhodin P.

Aus mehreren gleichartigen chromatographischen Läufen gewonnene stark angereicherte 1-OH-Cytorhodin-Chargen wurden vereinigt und erneut unter obigen Bedingungen chromatographiert. Aus ca. 80 mg wurden 20 mg reines 1-Hydroxy-Cytorhodin P erhalten.

1-OH-P: $^1$H-NMR
Absorptionsspektrum (nm)
a) 238 (4,58) 292 (3,77) 522 (4,03) 549 (3,92)
b) 237 (4,63) 296 (3,77) 522 (4,17) 549 (4,03) 561 (4,03)
c) 243 (4,65) 275 (3,85) 590 (4,11) 632 (4,17)
$C_{60}H_{82}N_2O_{22}$, M ber., 1182 (FAB-MS bestätigt).

Experimentelle Bedingungen:

1. Hochdruckflüssigkeitschromatographie (HPLC) an Kieselgel:

Stahlsäulen (4,6 × 250 mm), gepackt mit 7 µm LiChrosorb® Si 60 (Merck) oder 7 µ Kieselgel 60 (Grace); mobile Phase: $CHCl_3$-Methanol-86%ige Essigsäure-Triäthylamin-Wasser 80:10:10:2:0,01 Fluss: 0,5–1,5 ml/Min.; Nachweis bei 260 oder 490 nm mit Durchflussphotometer.

2. «reversed phase» HPLC
Stahlsäulen (4,6 × 250 mm) gepackt mit 10 μm Lichrosorb® RP-18 (Merck)
mobile Phase: $CHCl_3$-Methanol-10% Ammonacetat in Wasser 250:1050:375
Fluss: 0,5–1,5 ml/Min.; Nachweis bei 490 nm und 560 nm mit Durchflussphotometer.

Die verwendeten analytischen und präparativen HPLC-Säulen wurden i.a. mit den von der Fa. Merck (Darmstadt) empfohlenen Verfahren mit Hilfe einer pneumatischen Pumpe (Haskel) selbst gefüllt.

3. Präparative Säulenchromatographie
Zur üblichen präparativen Säulenchromatographie wurde 31 μm Kiselgel 60 (Gracc) oder 15–40 μm Kieselgel 60 (Merck) in offenen Glassäulen oder in unter Druck gepackten Stahlsäulen verwendet. Als mobile Phase dienten Lösungsmittelgemische ähnlicher Zusammensetzung wie das o.a. analytische HPLC System gegebenenfalls mit herabgesetzten Anteilen an Methanol, Essigsäure und Wasser.

Zur Chromatographie v.a. starker polarer Komponenten wurde 31 μm Kieselgel 60 (Gracc) mit 2 N HCl metallfrei gewaschen und nach Auswaschen der Säure in wässriger Suspension mit 5 N NaOH behandelt, bis sich ein pH von 7,5 eingestellt hatte. Nach Dekantieren wurde das modifizierte Kieselgel bei 130 °C getrocknet, gesiebt und mit der mobilen Phase in die Säule eingeschlämmt. Als mobile Phase wurden Lösungsmittelgemische $CHCl_3$/Wasser/Methanol 13:4:3–7 eingesetzt. Das Belastungsverhältnis betrug etwa 1:100–1:300.

4. Dünnschichtchromatographie (DC)
Kieselgelplatten $F_{254}$ (Merck) wurden entwikkelt mit System A: $CHCl_3$-Methanol-96% Essigsäure-Wasser-Triäthylamin 80:10:10:2:0,01 oder System B: $CHCl_3$-Methanol-99%ige Essigsäure 75:15:10:2.

5. Übliche Aufarbeitung
Die übliche Aufarbeitung wurde wie folgt ausgeführt: vereinigte Fraktionen aus «reversed phase»-HPLC, wurden mit etwa der Hälfte ihres Volumens an Wasser versetzt und anschliessend Chloroform bis zur Phasentrennung zugegeben. Die abgetrennte Chloroformphase wurde mit Wasser gewaschen, über wasserfreiem $Na_2SO_4$ getrocknet und i.V. eingedampft. Die so erhaltenen Produkte wurden auf folgende Weise von begleitenden Fetten, Weichmachern und Metallionen befreit: das Produkt - z.B. 30 mg - wurden in 20 ml Natrium-Acetatpuffer, pH 3,5 gelöst, mit 1 ml 0,001 M wässriger Äthylendiamintetraessigsäure (EDTA; mit NaOH auf pH 3,5 eingestellt) versetzt, und mit 5 ml Toluol geschüttelt. Die Toluolphase wurde verworfen, die wässrige Phase mit 2 N NaOH auf pH 7,5 eingestellt und mit 20 ml $CHCl_3$ unter gutem Schütteln extrahiert. Nach dem Trocknen über wasserfreiem $Na_2SO_4$ wurde filtriert und im Vakuum eingedampft. Gegebenenfalls wurde der Rückstand in wenig $CHCl_3$ gelöst, die Lösung durch eine Glasfritte abgesaugt und nach Zusatz von Heptan bis zur Trübung i.V. eingedampft.

6. Spektroskopische Untersuchungen
Die Identifizierung der Komponenten in den vorstehenden Beispielen erfolgte unter den anschliessend beschriebenen Messbedingungen:

Die Protonen-Resonanzspektren ($^1H$-NMR-Spektren) wurden auf einen HX-270 BRUKER Fourier-Transform-Kernresonanzspektrometer bei 270 MHZ gemessen. Die Konzentrationen betrugen 2–4 mg/0,5 ml 99,8% $CDCl_3$; die Lösungen wurden sofort nach Herstellung mit 0,1 ml 5%iger $Na_2CO_3$ in 99,5% $D_2O$ geschüttelt.

Die in den Abbildungen mit einem Stern versehenen Signale rühren her von niedermolekularen Verunreinigungen im ‰-Bereich und von Lösungsmittelresten.

Die Massenspektren wurden auf dem Massenspektrometer MS-902 S, AEI, unter Verwendung einer FAB-(Fast-Atom-Bombardment-) Ionenquelle gemessen. Die Substanzen wurden in einer Matrix von Thioglycerin in die Ionenquelle eingebracht, teilweise unter Zusatz von Ammoniumchlorid.

Die Absorptionsspektren wurden im Bereich 200–700 nm gemessen in:

a) Wasser-Methanol 1:9
b) 10% l n HCl in Methanol
c) 10% l n NaOH in Methanol.

Die Substanzkonzentration betrug 10–30 mg/l; angegeben werden die Absorptionsmaxima in nm und die molaren Extinktionskoeffizienten (log ε).

7. Ermittlung der zytotoxischen Aktivität
Die Bestimmung der zytostatischen Wirksamkeit der hier beschriebenen Verbindungen erfolgte an L1210-Leukämiezellen der Maus. Im einzelnen wurden folgende Testsysteme verwendet:

a) Proliferationsassay
Bei dieser Methode wird in vitro nach Inkubation der Zellen mit unterschiedlichen Konzentrationen der Testsubstanz ermittelt, inwieweit die Zellen radioaktive DNA-Vorläufer (z.B. C14 markiertes Thymidin) einbauen können. Unbehandelte L1210-Zellen werden den gleichen Testbedingungen unterworfen und dienen als Kontrolle. Im folgenden ist die Methode kurz beschrieben.

L1210-Zellen in exponentieller Wachstumsphase ($5 \times 10^3$/ml in RPMI 1640) werden in einer Mikrotiterplatte 72 Stunden mit unterschiedlichen Konzentrationen der Testsubstanz inkubiert (37°C, 5% $CO_2$, 95% relative Luftfeuchte). Kontrolle bestehen aus Zellen, die lediglich mit frischem Medium inkubiert werden. Alle Bestimmungen werden als 4fach-Bestimmungen durchgeführt. Nach 65 Stunden werden 50 μl C-1 14 Thymidin (1,5 μc/ml) zugegeben, um die DNA der Zelle radioaktiv zu markieren. Nach 7 Stunden Inkubation werden die Zellen abgesaugt, die DNA mit

5%iger Trichloressigsäure gefällt und nacheinander mit Wasser bzw. Methanol gewaschen.

Nach Trocknung bei 50°C wird die in die DNA eingebaute Radioaktivität nach Zugabe von 5 ml Szintillationsflüssigkeit ermittelt.

Die Ergebnisse werden angegeben als Verhältnis des Szintillationsindex nach Inkubation mit der Testsubstanz in Prozent der unbehandelten Kontrolle. Aus den so erhaltenen Messwerten wird die Dosiswirkungskurve ermittelt und graphisch die $IC_{50}$, d.h. die Konzentration, die unter Testbedingungen den Einbau von radioaktivem Thymidin um 50% gegenüber der Kontrolle erniedrigt, ermittelt. Die $IC_{50}$-Werte der hier beschriebenen Verbindungen im Vergleich zu Adriamycin (ADM) werden in der Tabelle 1 zusammengefasst.

b) Koloniebildung von L1210-Leukämiezellen in soft agar.

Diese Methode dient zum Nachweis eines Einflusses der Testsubstanzen auf das Wachstumsverhalten der Zellen über mehrere Generationen (bei einer Zellzykluszeit von 10–12 Stunden werden in der Testzeit von 7 Tagen ca. 14 aufeinanderfolgende Generationen beobachtet).

Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt:

500 Leukämiezellen pro Platte werden mit unterschiedlichen Konzentrationen von Testsubstanz 1 Stunde bei 37°C inkubiert. Anschliessend werden die Zellen zweimal mit McCoy5A Medium gewaschen und schliesslich in Petrischalen nach Zusatz von 0,3% Agar ausgegossen. Kontrollen werden lediglich mit frischem Medium inkubiert. Anstelle der 1stündigen Inkubation werden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37°C inkubiert (5% $CO_2$, 95% relative Luftfeuchtigkeit). Anschliessend wird die Anzahl der entstandenen Kolonien mit einem Durchmesser 60 µ gezählt. Die Ergebnisse werden angegeben als Koloniezahl in behandelten Agarplatten in Prozent der unbehandelten Kontrolle. Aus der so erhaltenen Dosiswirkungskurve wird die $IC_{50}$ als Mass für die Wirksamkeit der Substanz ermittelt. Die Ergebnisse für die hier beschriebenen Verbindungen im Vergleich zu Adriamycin (ADM) sind in der Tabelle 1 zusammengefasst.

Tabelle 1

| | Proliferationstest $IC_{50}$ (µg/ml) | Koloniebildungstest $IC_{50}$ (µg/ml) | |
|---|---|---|---|
| | | 7 Tage Inkubation | 1 Stunde Inkubation |
| ADM | $6{,}0 \times 10^{-3}$ | $2{,}2 \times 10^{-2}$ | $4{,}4 \times 10^{-2}$ |
| 1-OH-Cytorhodin A | $3{,}0 \times 10^{-3}$ | $2{,}8 \times 10^{-3}$ | $2{,}6 \times 10^{-2}$ |
| 1-OH-Cytorhodin V | $2{,}8 \times 10^{-3}$ | $4{,}5 \times 10^{-4}$ | $2{,}4 \times 10^{-3}$ |

Wie vorstehend ausgeführt, besitzen die erfindungsgemässen Verbindungen zytostatische Wirksamkeit, d.h. therapeutische Wirkung gegen Tumoren, insbesondere maligne Tumoren bei Tieren und Menschen.

Die Verbindung und die Säureadditionssalze können daher als Medikamente zur Behandlung von Tumoren verwendet werden. Die Verbindungen können auf verschiedene Weise in Abhängigkeit von der Dosierungsform verabreicht werden. Normalerweise werden die Verbindungen mit pharmazeutisch üblichen Trägerstoffen oder Verdünnungsmitteln vermischt verabreicht. So können sie z.B. einzeln oder in Mischung zusammen mit Trägerstoffen wie Maltose oder Lactose oder als nichttoxische Komplexe, z.B. als Desoxyribonukleinsäure-Komplex verabreicht werden.

Eine typische Verabreichungsart ist die Injektion einer Lösung der erfindungsgemässen Verbindungen in destilliertem Wasser oder in physiologischer Kochsalzlösung. Die Lösungen können intraperitoneal, intravenös oder intraarteriell injiziert werden.

Tagesdosis und Einheitsdosis können aus Tierversuchen und auch aus in vitro-Tests in der Weise festgesetzt werden, dass die Gesamtdosis, die kontinuierlich oder in Abständen verabreicht wird, einen vorher festgelegten Bereich nicht überschreitet. So beträgt die Gesamtdosis für einen Behandlungszyklus etwa 0,5–5 mg/kg Körpergewicht. Diese Dosis kann in entsprechenden Bruchteilen über einen Zeitraum von 7 Tagen verabreicht werden. Es ist jedoch klar, dass konkrete Dosen für die Behandlung von Mensch oder Tier individuell festgelegt werden können in Abhängigkeit von der jeweiligen Situation, z.B. Alter, Körpergewicht, Geschlecht, Empfindlichkeit, Nahrung, Zeitpunkt der Verabreichung, weiteren verabreichten Medikamenten, körperlichem Zustand der Patienten und Schwere der Erkrankung.

**Patentansprüche für die Vertragsstaaten: BE, CH, DE, FR, GB, IT, LI, LU, NL, SE**

1. Verbindungen der Formel I

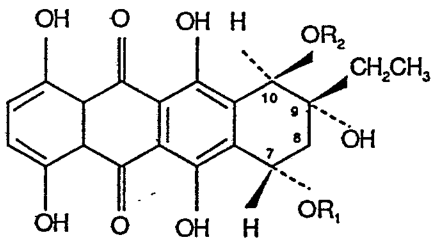

I

dadurch gekennzeichnet, dass
a) $R_1$ und $R_2$ gleich sind und jeweils die Zuckerkombination Roa-Rod-Rod oder Roa-dF=CinB darstellen, oder
b) $R_1$ und $R_2$ verschieden sind und jeweils die Zukkerkombination Roa-Rod-Rod und Roa-dF-Rod darstellen, oder
c) $R_1$ und $R_2$ verschieden sind, wobei $R_1$ die Zukkerkombination Roa-dF-CinA und $R_2$ die Zuckerkombination Roa-Rod-Rod oder Roa-Rod-Acu darstellt oder $R_1$ die Zuckerkombination Roa-Rod-Acu und $R_2$ die Zuckerkombination Roa-Rod-Rod darstellt,
sowie deren physiologisch unbedenklichen Säureadditionssalze.

2. Verfahren zur Herstellung von Verbindungen gemäss Anspruch 1, dadurch gekennzeichnet, dass der Mikroorganismus Streptomyces purpurascens (DSM 2658) bei erhöhter Sauerstoffversorgung auf sonst übliche Weise unter aeroben Bedingungen in Gegenwart eines Nährmediums fermentiert wird, und die gebildeten Verbindungen aus der Kulturflüssigkeit und dem Mycel durch Extraktion mit organischen Lösungsmitteln und anschliessende Chromatographie oder Verteilung in üblicher Weise isoliert werden.

3. Verfahren gemäss Anspruch 2, dadurch gekennzeichnet, dass der Mikroorganismus Streptomyces purpurascens DSM 2658 in einem Kohlenstoff- und Stickstoffquellen sowie anorganische Nährsalze und Spurenelemente enthaltenden Nährmedium bei Temperaturen von 24–40 °C und einen pH von 6,5–8,5 fermentiert wird, die gebildeten Verbindungen aus der Kulturflüssigkeit mit Ethylacetat und Chloroform und aus dem Mycel zunächst mit wässrigem Aceton, aus der abgetrennten wässrigen Phase sodann mit Ethylacetat extrahiert werden, die vereinigten Ethylacetatextrakte konzentriert und mit einem Acetatpuffer bei einem pH-Wert von 3,5 extrahiert werden, die wässrige Phase mit Toluol extrahiert wird und aus der verbleibenden wässrigen Lösung durch Chromatographie oder Verteilung die Verbindungen der Formel I isoliert werden.

4. Verfahren gemäss Anspruch 2, dadurch gekennzeichnet, dass die chromatographische Isolierung der Verbindungen der Formel I durch Kieselgel-Säulen, «reversed phase»-Adsorbentien, Tropfen-Gegenstrom-Chromatographie oder Verteilung und anschliessende Dünnschicht- oder Hochdruckflüssigkeitschromatographie erfolgt.

5. Arzneimittel, gekennzeichnet durch einen Gehalt an einer Verbindung gemäss Anspruch 1.

6. Verwendung einer Verbindung gemäss Anspruch 1 zur Herstellung eines Arzneimittels mit cytostatischer Wirkung.

**Patentansprüche für den Vertragsstaat: AT**

1. Verfahren zur Herstellung einer Verbindung der Formel I

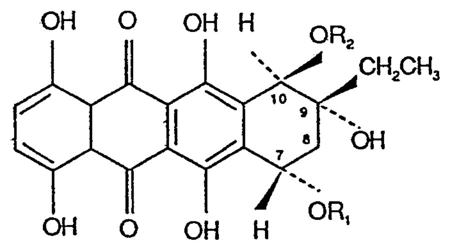

I

dadurch gekennzeichnet, dass
a) $R_1$ und $R_2$ gleich sind und jeweils die Zuckerkombination Roa-Rod-Rod oder Roa-dF=CinB darstellen, oder
b) $R_1$ und $R_2$ verschieden sind und jeweils die Zukkerkombination Roa-Rod-Rod und Roa-dF-Rod darstellen, oder
c) $R_1$ und $R_2$ verschieden sind, wobei $R_1$ die Zukkerkombination Roa-dF-CinA und $R_2$ die Zuckerkombination Roa-Rod-Rod oder Roa-Rod-Acu darstellt oder $R_1$ die Zuckerkombination Roa-Rod-Acu und $R_2$ die Zuckerkombination Roa-Rod-Rod darstellt,
sowie deren physiologisch unbedenklichen Säureadditionssalze, dadurch gekennzeichnet, dass der Mikroorganismus Streptomyces purpurascens (DSM 2658) bei erhöhter Sauerstoffversorgung auf sonst übliche Weise unter aeroben Bedingungen in Gegenwart eines Nährmediums fermentiert wird, und die gebildeten Verbindungen aus der Kulturflüssigkeit und dem Mycel durch Extraktion mit organischen Lösungsmitteln und anschliessende Chromatographie oder Verteilung in üblicher Weise isoliert werden.

2. Verfahren gemäss Anspruch 1, dadurch gekennzeichnet, dass der Mikroorganismus Streptomyces purpurascens DSM 2658 in einem Kohlenstoff- und Stickstoffquellen sowie anorganische Nährsalze und Spurenelemente enthaltenden Nährmedium bei Temperaturen von 24–40 °C und einen pH von 6,5–8,5 fermentiert wird, die gebildeten Verbindungen aus der Kulturflüssigkeit mit Ethylacetat und Chloroform und aus dem Mycel zunächst mit wässrigem Aceton aus der abgetrennten wässrigen Phase sodann mit Ethylacetat extrahiert werden, die vereinigten Ethylacetatextrakte konzentriert und mit einem Acetatpuffer bei einem pH-Wert von 3,5 extrahiert werden, die wässrige Phase mit Toluol extrahiert wird und aus der verbleibenden wässrigen Lösung durch Chromatographie oder Verteilung die Verbindungen der Formel I isoliert werden.

3. Verfahren gemäss Anspruch 1, dadurch gekennzeichnet, dass die chromatographische Isolierung der Verbindungen der Formel I durch Kieselgel-Säulen, «reversed phase»-Adsorbentien, Tropfen-Gegenstrom-Chromatographie oder Verteilung und anschliessende Dünnschicht-oder Hochdruckflüssigkeitschromatographie erfolgt.

4. Verwendung einer Verbindung gemäss Anspruch 1 zur Herstellung eines Arzneimittels mit cytostatischer Wirkung.

**Claims for the Contracting States: BE, CH, DE, FR, GB, IT, LI, LU, NL, SE**

1. Compounds of the formula I

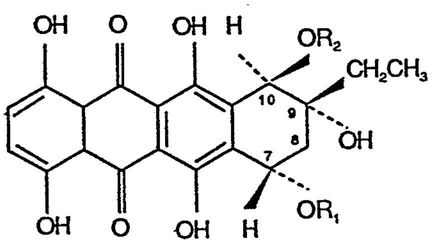

I

wherein
a) $R_1$ and $R_2$ are identical and each represent the sugar combination Roa-Rod-Rod or Roa-dF=CinB, or
b) $R_1$ and $R_2$ are different and each represent the sugar combination Roa-Rod-Rod and Roa-dF-Rod, or
c) $R_1$ and $R_2$ are different, $R_1$ representing the sugar combination Roa-dF-CinA and $R_2$ representing the sugar combination Roa-Rod-Rod or Roa-Rod-Acu, or $R_1$ representing the sugar combination Roa-Rod-Acu and $R_2$ representing the sugar combination Roa-Rod-Rod

and their physiologically acceptable acid addition salts.

2. A process for the preparation of compounds as claimed in claim 1, which comprises fermenting the microorganism Streptomyces purpurascens (DSM 2658) in the presence of a nutrient medium with an elevated oxygen supply, otherwise in a customary manner under aerobic conditions, and isolating the compounds formed from the culture liquid and the mycelium by extraction with organic solvents followed by chromatography or partition in a customary manner.

3. The process as claimed in claim 2, wherein the microorganism Streptomyces purpurascens DSM 2658 is fermented in a nutrient medium containing sources of carbon and nitrogen as well as inorganic nutrient salts and trace elements, at temperatures of 24–40 °C and a pH of 6.5–8.5, the compounds formed are extracted from the culture liquid using ethyl acetate and chloroform, and from the mycelium initially with aqueous acetone and then from the separated aqueous phase using ethyl acetate, the combined ethyl acetate extracts are concentrated and extracted with an acetate buffer at a pH of 3.5, the aqueous phase is extracted with toluene, and the compounds of the formula I are isolated from the remaining aqueous solution by chromatography or partition.

4. The process as claimed in claim 2, wherein the chromatographic isolation of the compounds of the formula I is carried out using silica gel columns, reversed phase adsorbents, droplet countercurrent chromatography or partition, followed by thin-layer or high-pressure liquid chromatography.

5. A medicament containing a compound as claimed in claim 1.

6. The use of a compound as claimed in claim 1 for the preparation of a medicament having a cytostatic action.

**Claims for the Contracting State: AT**

1. A process for the preparation of a compound of the formula I

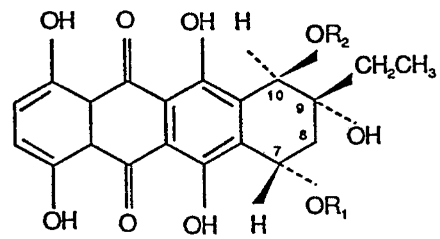

I

wherein
a) $R_1$ and $R_2$ are identical and each represent the sugar combination Roa-Rod-Rod or Roa-dF=CinB, or
b) $R_1$ and $R_2$ are different and each represent the sugar combination Roa-Rod-Rod and Roa-dF-Rod, or
c) $R_1$ and $R_2$ are different, $R_1$ representing the sugar combination Roa-dF-CinA and $R_2$ representing the sugar combination Roa-Rod-Rod or Roa-Rod-Acu, or $R_1$ representing the sugar combination Roa-Rod-Acu and $R_2$ representing the sugar combination Roa-Rod-Rod,

and their physiologically acceptable acid addition salts, which comprises fermenting the microorganism Streptomyces purpurascens (DSM 2658) in the presence of a nutrient medium with an elevated oxygen supply, otherwise in a customary manner under aerobic conditions, and isolating the compounds formed from the culture liquid and the mycelium by extraction with organic solvents followed by chromatography or partition in a customary manner.

2. The process as claimed in claim 1, wherein the microorganism Streptomyces purpurascens DSM 2658 is fermented in a nutrient medium containing sources of carbon and nitrogen as well as inorganic nutrient salts and trace elements, at temperatures of 24–40 °C and a pH of 6.5–8.5, the compounds formed are extracted from the culture liquid using ethyl acetate and chloroform, and from the mycelium initially with aqueous acetone and then from the separated aqueous phase using ethyl acetate, the combined ethyl acetate extracts are concentrated and extracted with an acetate buffer at a pH of 3.5, the aqueous phase is extracted with toluene, and the compounds of the formula I are isolated from the remaining aqueous solution by chromatography or partition.

3. The process as claimed in claim 1, wherein

the chromatographic isolation of the compounds of the formula I is carried out using silica gel columns, reversed phase adsorbents, droplet countercurrent chromatography or partition, followed by thin-layer or high-pressure liquid chromatography.

4. The use of a compound as claimed in claim 1 for the preparation of a medicament having a cytostatic action.

**Revendications pour les Etats Contractants: BE, CH, DE, FR, GB, IT, LI, LU, NL, SE**

1. Composés de formule I

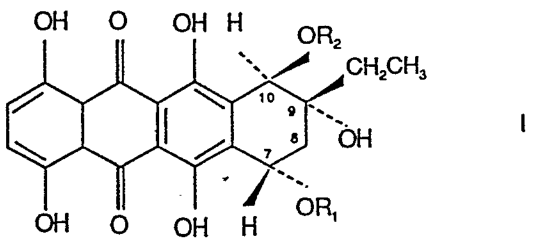

I

caractérisés en ce que
a) $R_1$ et $R_2$ sont identiques et représentent chacun la combinaison de sucres Roa-Rod-Rod ou Roa-dF=CinB, ou
b) $R_1$ et $R_2$ sont différents et représentent respectivement la combinaison de sucres Roa-Rod-Rod et Roa-dF-Rod, ou
c) $R_1$ et $R_2$ sont différents, $R_1$ représentant la combinaison de sucres Roa-dF-CinA et $R_2$, la combinaison de sucres Roa-Rod-Rod ou Roa-Rod-Acu, ou $R_1$ représentant la combinaison de sucres Roa-Rod-Acu et $R_2$, la combinaison de sucres Roa-Rod-Rod,

et sels d'addition avec des acides physiologiquement acceptables de ceux-ci.

2. Procédé pour la préparation de composés selon la revendication 1, caractérisé en ce que l'on cultive par fermentation le micro-organisme Streptomyces purpurascens (DSM 2658) avec apport élevé d'oxygène, de façon par ailleurs usuelle dans des conditions aérobies, en présence d'un milieu nutritif, et, à partir du milieu de culture et du mycélium, on isole à la manière habituelle les composés formés, par extraction avec des solvants organiques et chromatographie ou partage subséquent.

3. Procédé selon la revendication 2, caractérisé en ce que l'on cultive par fermentation le micro-organisme Streptomyces purpurascens DSM 2658 dans un milieu nutritif contenant des sources de carbone et d'azote ainsi que des sels minéraux nutritifs et des oligoéléments, à des températures de 24 à 40°C et à un pH de 6,5-8,5, on extrait les composés formés, à partir du liquide de culture, avec de l'acétate d'éthyle et du chloroforme, et à partir du mycélium, d'abord avec de l'acétone en solution aqueuse, puis, à partir de la phase aqueuse séparée, avec de l'acétate d'éthyle, on concentre les extraits d'acétate d'éthyle réunis et on extrait la solution résultante avec un tampon acétate à un pH de 3,5, on extrait la phase aqueuse avec du toluène, et à partir de la solution aqueuse résiduelle, on isole par chromatographie ou partage les composés de formule I.

4. Procédé selon la revendication 2, caractérisé en ce que l'on effectue l'isolement chromatographique des composés de formule I par chromatographie sur gel de silice, sur adsorbants en phases inversées, chromatographie de gouttes à contre-courant, ou par partage et chromatographie sur couche mince ou chromatographie liquide à haute pression subséquente.

5. Médicament caractérisé par une teneur en un composé selon la revendication 1.

6. Utilisation d'un composé selon la revendication 1 pour la fabrication d'un médicament à action cytostatique.

**Revendications pour l'Etat contractant: AT**

1. Procédé pour la préparation d'un composé de formule I

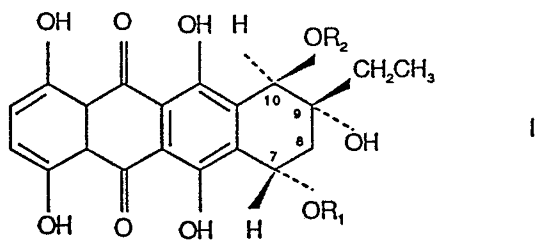

I

caractérisé en ce que
a) $R_1$ et $R_2$ sont identiques et représentent chacun la combinaison de sucres Roa-Rod-Rod ou Roa-dF=CinB, ou
b) $R_1$ et $R_2$ sont différents et représentent respectivement la combinaison de sucres Roa-Rod-Rod et Roa-dF-Rod, ou
c) $R_1$ et $R_2$ sont différents, $R_1$ représentant la combinaison de sucres Roa-dF-CinA et $R_2$, la combinaison de sucres Roa-Rod-Rod ou Roa-Rod-Acu, ou $R_1$ représentant la combinaison de sucres Roa-Rod-Acu et $R_2$, la combinaison de sucres Roa-Rod-Rod,

ainsi que des sels d'addition avec des acides physiologiquement acceptables de celui-ci, caractérisé en ce que l'on cultive par fermentation le micro-organisme Streptomyces purpurascens (DSM 2658) avec apport élevé d'oxygène, de façon par ailleurs usuelle dans des conditions aérobies, en présence d'un milieu nutritif, et, à partir du milieu de culture et du mycélium, on isole à la manière habituelle les composés formés, par extraction avec des solvants organiques et chromatographie ou partage subséquent.

2. Procédé selon la revendication 1, caractérisé en ce que l'on cultive par fermentation le micro-organisme Streptomyces purpurascens DSM 2658 dans un milieu nutritif contenant des sources de carbone et d'azote ainsi que des sels minéraux nutritifs et des oligoéléments, à des températures de 24 à 40°C et à un pH de 6,5-8,5, on extrait les composés formés, à partir du liquide de culture, avec de l'acétate d'éthyle et du chloroforme, et à partir du mycélium, d'abord avec de l'acétone en solution aqueuse, puis, à partir de la phase aqueuse séparée, avec de l'acétate d'éthyle, on concentre les extraits d'acétate d'éthyle réunis et on extrait la solution résultante avec un tampon acétate à un pH de 3,5, on extrait la phase aqueuse avec du toluène, et à partir de la solution aqueuse résiduelle, on isole par chromatographie ou partage les composés de formule I.

3. Procédé selon la revendication 1, caractérisé en ce que l'on effectue l'isolement chromatographique des composés de formule I par chromatographie sur gel de silice, sur adsorbants en phases inversées, chromatographie de gouttes à contre-courant, ou par partage et chromatographie sur couche mince ou chromatographie liquide à haute pression subséquente.

4. Utilisation d'un composé selon la revendication 1 pour la fabrication d'un médicament à action cytostatique.

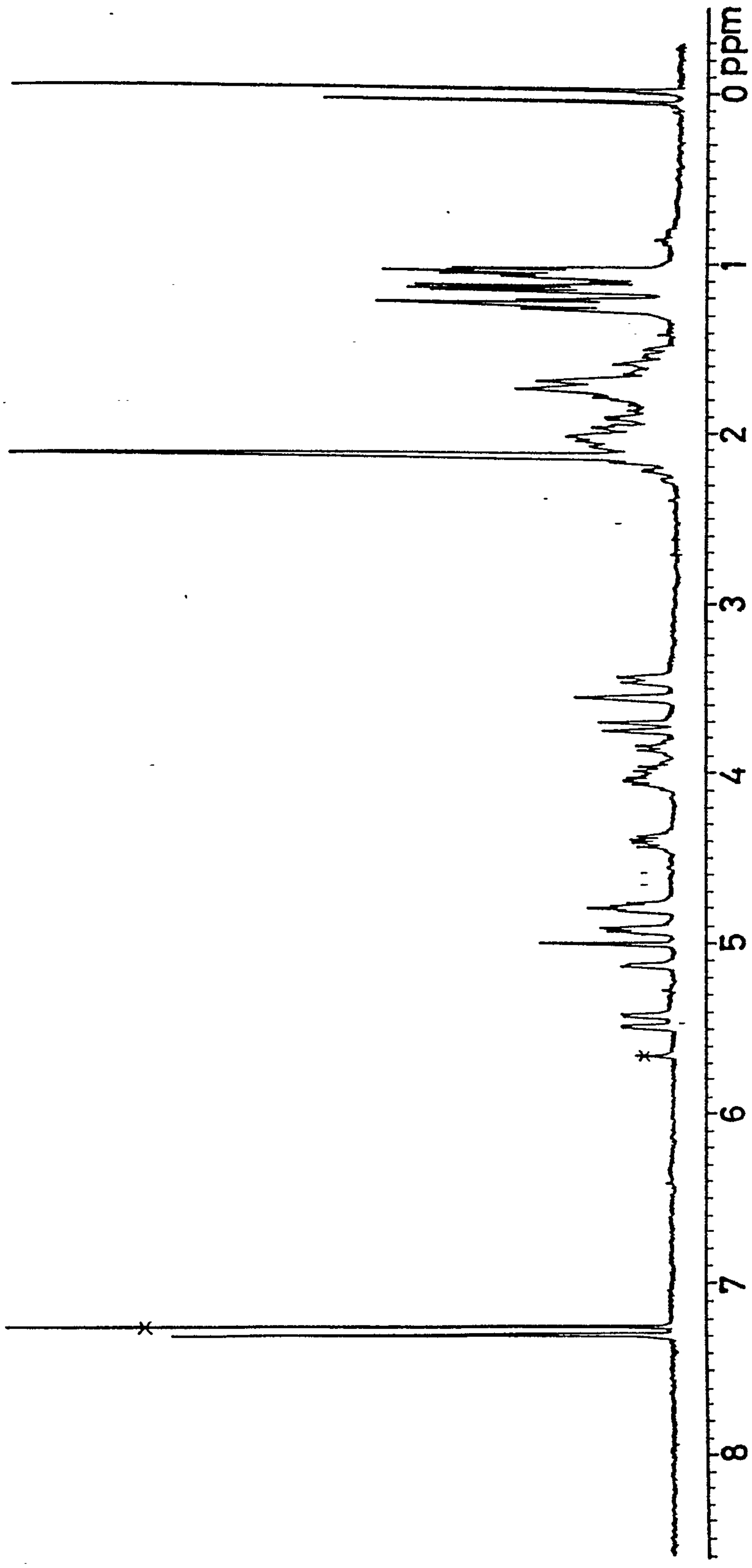
0 ppm
1
2
3
4
5
6
7
8